# EUROPÄISCHE PATENTSCHRIFT

(11) **EP 2 773 421 B1**
(45) Veröffentlichungstag und Bekanntmachung des Hinweises auf die Patenterteilung: **23.11.2016**
(21) Anmeldenummer: 12798595.0
(22) Anmeldetag: 08.10.2012
(51) Int. Cl.: A61N 1/05, A61M 25/10

(54) **BALLON KATHETER**
BALLOON CATHETER
CATHÉTER À BALLONNET

(30) Priorität: 05.11.2011 DE 202011107537 U
(43) Veröffentlichungstag der Anmeldung: 10.09.2014
(73) Patentinhaber: Osypka, Peter, 79639 Grenzach-Wyhlen (DE)
(72) Erfinder: Osypka, Peter, 79639 Grenzach-Wyhlen (DE)
(86) Internationale Anmeldenummer: PCT/DE2012/000980
(87) Internationale Veröffentlichungsnummer: WO 2013/064132

(56) Entgegenhaltungen:
- WO-A2-95/09015
- DE-A1- 19 626 181
- US-A- 4 706 688
- US-A- 5 056 532

## Beschreibung

Die Erfindung betrifft einen ösophagealen Ballon Katheter, der zur Stimulation, Kardioversion bei Vorhofflimmern und Defibrillation des Herzens, eingesetzt werden kann.

### Problem:

Postoperatives Vorhofflimmern tritt relativ häufig auf und ist einer der wichtigsten Gründe für die postoperative Morbidität. Insgesamt scheint Vorhofflimmern nach Herzoperationen in den letzten Jahren zugenommen zu haben, was mit einem immer älter werdenden Patientenkollektiv erklärt wird. Literaturangaben über postoperatives Vorhofflimmern bei Patienten mit vorbestehendem Sinusrhythmus ergaben ein durchschnittliches Auftreten in 30%-40% nach Bypass Operationen am Herzen. Vorhofflimmern führt zu einer schnellen Überleitung der Erregung auf die Ventrikel, so dass es zu akuter hämodynamischer Instabilität kommen kann. Die elektrische Kardioversion ist eine nicht-medikamentöse und sehr effektive Methode zur Wieder-herstellung des Sinus Rhythmus, die allerdings eine Kurznarkose zur Voraussetzung hat. Diese Kurznarkose kann jedoch gerade bei Patienten nach Bypass Operation die bestehenden neuronalen Probleme (Vigilanz) durch die soeben überstandene Herzoperation verschärfen, was zu einer verlängerten Aufwachphase führen kann oder sogar eine erneute Intubation mit maschineller Beatmung erforderlich machen. Ein weiteres Problem stellt die Antikoagulation bei postoperativen Patienten mit Vorhofflimmern dar. Wenn die Rhythmusstörung länger als 24 Stunden anhält, ist eine Antikoagulation erforderlich, um die Bildung von Thromben mit der Gefahr eines Schlaganfalls zu reduzieren. All diese Faktoren führen zu einem komplizierten postoperativen Verlauf bei Patienten nach Bypass Operation, der sich in einem um ca. 5 Tage verlängerten Krankenhausaufenthalt mit vermehrten Kosten nieder-schlägt. Bei Herzkammerflimmern ist es von grosser Wichtigkeit, dass der DEFI-Schock so schnell wie möglich appliziert wird. Das geschieht z. Z. durch Auflegen von grossflächigen Elektroden auf die Brustfläche, die sich über dem Herzen befindet. Die abgegebene Energiemenge beträgt dabei zwischen 200 und 300 Joule.

### Wie wird das Problem bisher gelöst:

Für die temporäre Stimulation des Herzens über den Ösophagus sind derartige Elektroden seit vielen Jahren bekannt und werden routinemässig eingesetzt. Die Beseitigung von Vorhof- bzw. Kammer-Flimmern nach einer Herz Operation oder anderen krankhaften Ursachen des Herzens erfolgt bisher durch einen äusseren elektrischen Energieimpuls mittels eines Defibrillators, appliziert durch Auflage oder Aufkleben von grossflächigen Elektroden auf den Brustkorb des Patienten. Beim Vorhofflimmern ist es notwendig, dass beim Patienten vorher eine Ultraschall-untersuchung des linken Herzohres durchgeführt werden muss und während der Kardioversion eine Narkose notwendig ist. Diese Massnahmen verlängern den Aufenthalt des Patienten auf der Intensivstation um etwa 2-4 Tage. Bei der Cardio Resynchronisation Therapie (CRT) ist die Einstellung der Impulsverzögerung zwischen den beiden Ventrikel-Stimulationsimpulsen von grosser Wichtigkeit zur optimalen Einstellung der Herzleistung. Auch hier sind Ösophagus Elektroden inzwischen von grosser Hilfe. Bei allen Anwendungen von Elektroden im Ösophagus ist es wichtig, dass diese möglichst nahe am Herzen liegen. Ohne ein Röntgengerät auf der Intensivstation ist die Position bzw. Ausrichtung des Ösophagus Katheters oft sehr schwierig. Bei der Therapie von Vorhofflimmern mittels RF-Ablation, wäre eine Kühlung des Ösophagus wünschenswert:

US 5 056 532 A offenbart einen mehrpoligen Elektroden-Katheter zum Einsatz in der Speiseröhre.

Es besteht daher die Aufgabe, einen Ösophagus Katheter zu schaffen, wobei die Elektroden möglichst nahe am Herzen liegen, insbesondere nahe am linken Vorhof und der Ösophagus Katheter eine unmittelbare, schnelle und leicht zugängliche Anwendung der unterschiedlichen diagnostischen und therapeutischen Anwendungen am Herzen ermöglicht. Da der Ösophagus anatomisch direkt hinter dem linken Vorhof und Teile des Ventrikels verläuft, und der Katheter ohne Narkose durch einfaches Schlucken eingeführt werden kann, bietet er sich für diese Anwendungen an. Diese Aufgabe wird mit den Mitteln und Merkmalen des Patentanspruches 1 gelöst.

### Abbildungen:

Weitere Einzelheiten, Merkmale und Vorteile der Erfindung lassen sich im folgenden Beschreibungsteil entnehmen, in dem anhand der Zeichnungen und Ausführungsbeispiele die Erfindung näher erläutert wird.

Sie zeigen in schematischer Darstellung in:
Fig. 1 die anatomische Lage von Herz und Ösophagus im menschlichen Körper.
Fig. 2 eine beispielhafte Ausführungsform eines Ösophagus Ballon Katheters für die Kardioversion und Stimulation des Herzens, dessen Elektroden mit Hilfe des Ballons in Richtung des Herzens wandständig ausgerichtet werden können.
Fig. 3 eine beispielhafte Ausführungsform eines Ösophagus Ballon Katheters für die Kardioversion und Stimulation des Herzens, dessen Pole in ihrem Abstand veränderbar sind.
Fig. 4 eine beispielhafte Ausführungsform eines Ösophagus Katheters für die Kardioversion und Stimulation des Herzens, bestehend aus zwei Ballons, deren Pole in ihrem Abstand veränderbar sind und in Richtung Herz wandständig ausgerichtet werden können.
Fig. 5 eine beispielhafte Ausführungsform eines Ösophagus Ballon Katheters für die Kardioversion und Stimulation des Herzens, der zusätzlich zu den Polen mit Magneten versehen ist.
Fig. 6 eine beispielhafte Ausführungsform eines Ösophagus Katheters für die Kardioversion und Stimulation des Herzens, bestehend aus einem Ballon oder zwei Ballons. Der Katheter weist mindestens einen Hohlkanal auf, durch den ein weiterer Katheter eingeführt werden kann.
Fig. 7 eine beispielhafte Ausführungsform eines Ösophagus Ballon Katheters für die Kardioversion und Stimulation des Herzens, dessen Pole in Bahnen verlaufen, in ihrem Abstand veränderbar sind und mit dem Ballon in Richtung Herz wandständig ausgerichtet werden können.
Fig. 8 eine beispielhafte Ausführungsform des proximalen Teils des Ösophagus Ballon Katheters.
Fig. 9 eine beispielhafte Ausführungsform eines Ösophagus Ballon Katheters für die Kardioversion und Stimulation des Herzens mit zusätzlichen zwei über den Vorhöfen platzierten Aussenelektroden.
Fig. 10 eine beispielhafte Ausführungsform eines Ösophagus Ballon Katheters für die Defibrillation und Stimulation des Herzens mit einer zusätzlichen grossflächigen über den Herzkammern platzierten Aussenelektrode.

### Beschreibung der Abbildungen:

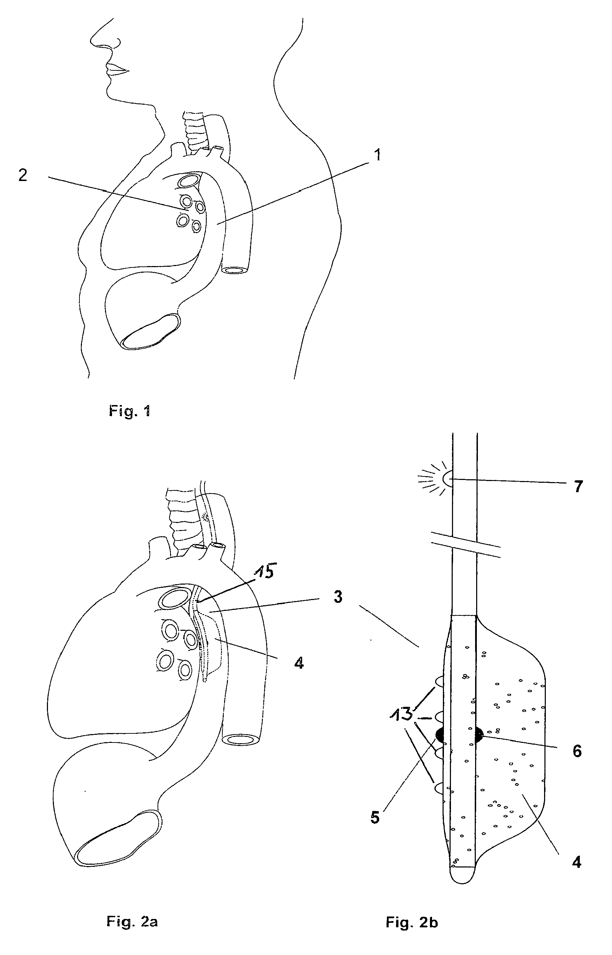

Fig. 1 zeigt die anatomische Lage von Herz und Ösophagus im menschlichen Körper. Es ist gut zu erkennen, dass der Ösophagus (1) anatomisch nahe am linken Vorhof des Herzens (2) liegt.

Fig. 2a zeigt eine beispielhafte Ausführungsform eines mehrpoligen Ösophagus Elektroden-Katheters. Der Katheter weist einen flexiblen Schaft (15) mit einem proximalen Ende und einem distalen Ende und eine mehrpolige Elektrode (3) auf und ist dadurch gekennzeichnet, dass das distale Ende des Katheters als einseitig angeordneter aufblasbarer Ballon (4) ausgebildet ist, derart dass die mehrpolige Elektrode (3) mit Hilfe des Ballons in Richtung des Herzens wandständig ausgerichtet ist.

Fig 2b: Man erkennt die Pole (13). Der Ballon kann mit Kühlflüssigkeit gefüllt werden und enthält einen Temperatur Sensor (6). Ein weiterer Temperatur Sensor (5) misst die Temperatur im Ösophagus. Eine rote Leuchtdiode (LED) (7), die im definierten Abstand vom Ballon angeordnet ist und bei der Anwendung im Kehlkopf Bereich von aussen sichtbar ist, ist eine Hilfe bei der Position des Ballons.

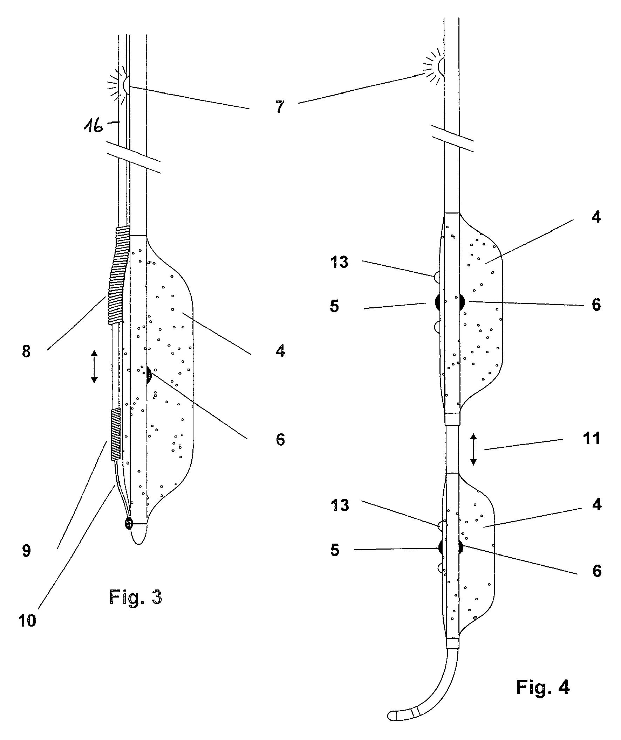

Fig. 3 zeigt eine beispielhafte Ausführungsform einer bipolaren Ösophagus Ballon Elektrode für die Kardioversion und Stimulation des Herzens in Form von zwei Metallwendeln (8,9). Die Wendeln können in ihrer Grösse und in ihrem Abstand je nach Anatomie des Patienten unterschiedlich konfiguriert sein. Der Katheter weist einen weiteren Schaft (16) auf, der den proximalen Pol (8) der Elektrode trägt, während der distale Pol (9) im inneren des Schafts (16) über einen Führungsdraht (10) verschiebbar angeordnet ist.

Während der proximale Pol (8) der Elektrode im äusseren Schaft (16) untergebracht ist, kann der distale Pol (9) der Elektrode, entsprechend den anatomischen Gegebenheiten des Patienten, als separater Schaft im inneren des Aussenschlauches verschoben werden.

Der Führungsdraht ist distal mit dem Katheter Schaft verbunden. Gleichzeitig ist dort ein Temperaturfühler angebracht. Temperaturfühler können je nach Kundenwunsch an mehreren Teilen des Katheters angebracht werden, so z. B. im Inneren des Ballons oder an äusseren Teilen des Ballons. Die mechanische Fixierung der beiden Pol-Schäfte erfolgt nach optimaler Positionierung proximal mit einem Schraubventil (hier nicht dargestellt).

Der elektrische Zuleitungswiderstand im Katheter zu den Elektroden Polen sollte möglichst gering sein. Als Wendelwerkstoff kommen die bekannten biokompatiblen Materialien, wie z. B. Edelstahl und seine Legierungen, Platin, Gold, Silber, Wolfram mit optionalen leitfähigen Beschichtungen zur Anwendung. Wird der Ballon (4) mit Flüssigkeit oder Gas gefüllt, wobei die Pole in Richtung des Herzens ausgerichtet sind, so werden diese automatisch gegen die Wand des Ösophagus gedrückt und damit gelangen sie in die Nähe des linken Vorhofs, was wiederum zu einer Reduzierung der notwendigen elektrischen Energie für die Kardioversion bzw. Stimulation führt. Die Eigenschaften des Ballons können auch bei entsprechender Füllung als Kühlvorrichtung für den Ösophagus z. B. bei der Hochfrequenz Ablation verwendet werden. Durch Integration von Sensoren (6), kann auch damit die Temperaturüberwachung durchgeführt werden.

Fig. 4 zeigt eine beispielhafte Ausführungsform einer Ösophagus Ballon Elektrode, deren Pole (13) jeweils auf einem separaten Ballon (4) angeordnet sind. Die Anordnung kann identisch sein wie in Fig. 4 dargestellt. Es ist aber auch eine unterschiedliche Anordnung denkbar. Der Abstand (11) zwischen den Ballons ist veränderbar. Die Elektroden sind durch die Ballons in Richtung Herz wandständig ausgerichtet. Damit kann man den Katheter optimal an die anatomischen Gegebenheiten anpassen. Das ist besonders günstig, wenn man den Katheter für die Vorhof Kardioversion oder Defibrillation benutzt. Neben der Anzahl der Pole pro Ballon, können auch hier zusätzliche Temperatur Sensoren (5 , 6) angebracht werden.

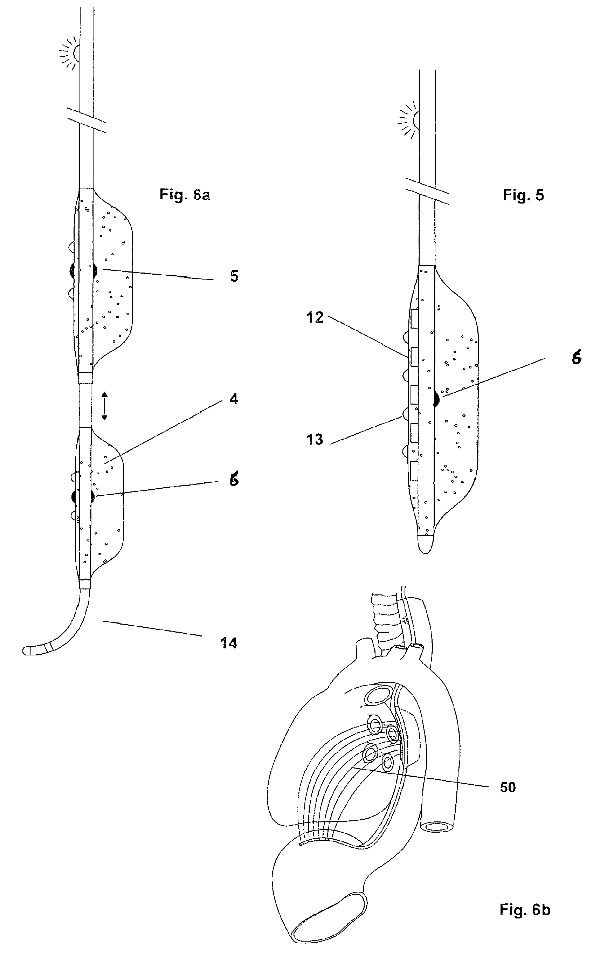

Fig. 5 zeigt eine beispielhafte Ausführungsform einer Ösophagus Ballon Elektrode für die Kardioversion und Stimulation des Herzens, die zusätzlich mit Magneten (12) versehen ist. Die Magnete dienen dazu, Führungsdrähte, die distal mit einem entsprechenden Magneten ausgestattet sind und sich im linken Vorhof befinden, zu fixieren. Über diese Führungsdrähte lassen sich dann sehr einfach z. B. entsprechende Ballon Katheter zur Beseitigung von Vorhofflimmern in den linken Vorhof einführen, die man insbesondere mit einem gewissen Druck gegen die Ausgänge der Lungenvenen drücken kann, um so die gewünschten gleichmässigen Bahnen der Ablation zu verwirklichen.

Fig. 6a zeigt eine beispielhafte Ausführungsform einer Ösophagus Ballon Elektrode für die Kardioversion und Stimulation des Herzens, die mindestens einen Hohlkanal aufweist, durch den ein weiterer Elektroden Katheter (14) eingeführt werden kann. Besonders vorteilhaft ist ein derartiger Katheter bei einer Defibrillation des gesamten Herzens, wenn er zusätzlich steuerbar ist und so im Magen unter die Herzspitze positioniert werden kann. Fig. 6b zeigt beispielsweise die Feldstärkeverteilung (50) einer derartigen Anordnung zum Defibrillieren bei Herzkammerflimmern. Hier werden durch den Elektrischen Schock optimal alle Areale des Herzens erfasst.

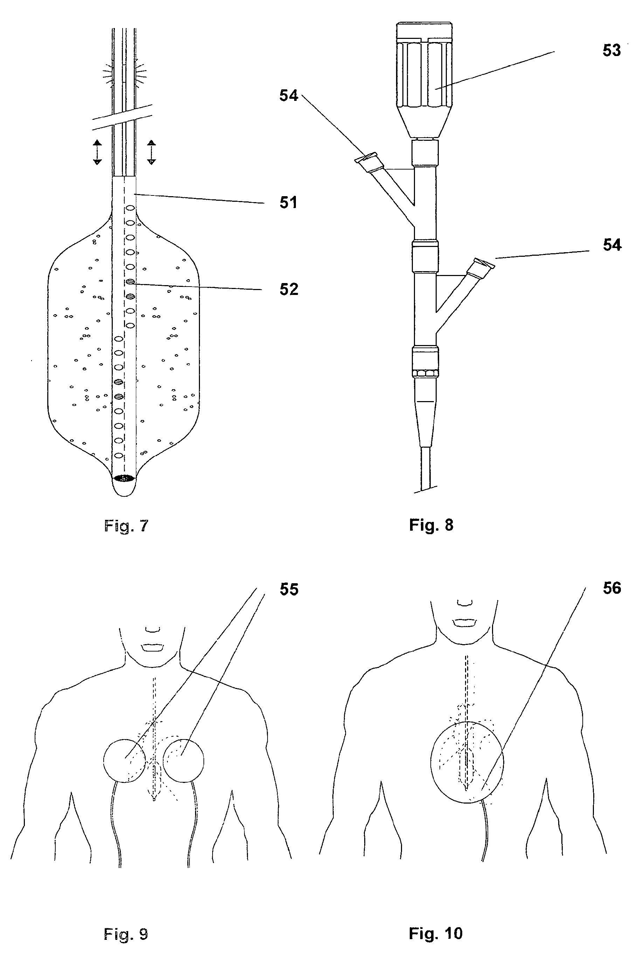

Fig. 7 zeigt eine beispielhafte Ausführungsform einer Ösophagus Ballon Elektrode, deren Pole in zwei voneinander isolierten Bahnen (51) mit kleinen Öffnungen (52) versehen sind, in ihrem Abstand veränderbar sind und die mit dem Ballon wandständig gegen das Herz ausgerichtet werden können. Die Polbahnen können aus einem oder mehreren Metallstreifen bestehen oder auch, ähnlich wie bei gedruckten Platinen, aus mehreren übereinander isolierten metallbeschichteten leitfähigen Bahnen aus Kunststoff (z. B. Polyimid-Kapton) oder Metall zusammengesetzt sein, sodass Pole in beliebiger Grösse, Form und Abstand realisiert werden können. Je nach Anwendung kann die Anordnung der Pole auf den Bändern individuell gestaltet werden. Durch das unabhängige Verschieben der Bänder ergeben sich eine Vielzahl von Pol Positionen.

Durch das Verschieben der flexiblen Bahnen und durch Füllen des Ballons kann jedes Elektrodenpaar je nach den anatomischen Gegebenheiten des Herzens wandständig positioniert werden. Derartige Leiterbahnen können neben elektrischen Polen für die Stimulation, Defibrillation oder Hochfrequenz Ablation auch Sensoren zur Temperaturmessung oder Ultraschallkristalle sowie optische Messeinrichtungen enthalten. Auch die isolierten Bahnen, auf denen sich die Pole befinden, können in Form, Grösse und Anzahl verändert werden.

Fig. 8 zeigt eine beispielhafte Ausführungsform des proximalen Teils des Ösophagus Katheters, nämlich den Stecker (53), mit der entsprechenden Anzahl der Pole und die notwendigen Einführungen (54) zur Füllung der Ballons.

Fig. 9 zeigt eine beispielhafte Ausführungsform einer Ösophagus Ballon Elektrode für die Kardioversion und Stimulation des Herzens mit zwei über den Vorhöfen platzierten Aussenelektroden (55). Mit dieser Anordnung kann ebenfalls die Terminierung von Vorhofflimmern durchgeführt werden. Der elektrische Schock wird dabei zwischen den Polen des Ballons und den beiden parallel geschalteten Aussenelektroden appliziert.

Fig. 10 zeigt eine beispielhafte Ausführungsform einer Ösophagus Ballon Elektrode für die Defibrillation und Stimulation des Herzens mit einer grossflächigen über den Herzkammern (56) platzierten Aussenelektrode. Mit dieser Anordnung kann die Terminierung von Herzkammer Flimmern vorgenommen werden. Der elektrische Schock wird dabei zwischen den Polen des Ballons und der Aussenelektrode appliziert.

## Patentansprüche

1. Mehrpoliger Ösophagus Elektroden-Katheter, der zur Überwachung, Stimulation und Kardioversion bei Vorhofflimmern des Herzens eingesetzt werden kann, wobei der Katheter einen flexiblen Schaft (15) mit einem proximalen Ende und einem distalen Ende aufweist, wobei das distale Ende des Katheters als einseitig angeordneter aufblasbarer Ballon (4) ausgebildet ist, **dadurch gekennzeichnet, dass** der Katheterzwei aufblasbare Ballons (4) aufweist, wobei auf jedem Ballon eine mehrpolige Elektrode (3) angeordnet ist, derart, dass die mehrpolige Elektrode (3) mit Hilfe des Ballons in Richtung des Herzens wandständig ausgerichtet werden kann und dass der Abstand (11) zwischen den Ballons (4) veränderbar ist.

2. Katheter nach Anspruch 1, **dadurch gekennzeichnet, dass** die Ballons mit Gas und/oder einer Flüssigkeit gefüll sind.

3. Katheter nach Anspruch 2, **dadurch gekennzeichnet, dass** die Flüssigkeit eine Kühlflüssigkeit ist.

4. Katheter nach Anspruch 1, **dadurch gekennzeichnet, dass** der Schaft (15) einen Hohlkanal aufweist durch den ein weiterer Katheter (14) eingeführt werden kann.

5. Katheter nach einem der Ansprüche 1-4, **dadurch gekennzeichnet, dass** der Katheter mindestens einen Temperatursensor (5) aufweist, der die Temperatur im Ösophagus misst, und gegebenenfalls einen weiteren Temperatursensor (6) aufweist.

6. Katheter nach einem der Ansprüche 1-5, **dadurch gekennzeichnet, dass** der Katheter auf der dem Herzen zugewandten Seite mindestens einen Magneten (12) enthält.

7. Katheter nach einem der Ansprüche 1-6, **dadurch gekennzeichnet dass** auf dem Schaft (15) parallel in der gleichen Elektroden Ebene mindestens eine Leuchtdiode (LED) (7) angebracht ist.

8. Mehrpoliger Ösophagus Elektroden-Katheter gemäss einem der Ansprüche 1-7 zur Überwachung, Stimulation und Kardioversion bei Vorhofflimmern des Herzens.

## Claims

1. Multipolar esophageal electrode catheter device that can be used for monitoring, stimulation, and cardioversion when treating atrial fibrillation, said catheter having a flexible shaft (15) with a proximal end and a distal end, whereby the distal end of the catheter is formed as an inflatable balloon (4) said balloon being arranged on one side, **characterized in that** the catheter device has two inflatable balloons (4), whereby a multipole electrode (3) is arranged on each balloon such that the multipolar electrode (3) can be aligned appropriately in the direction of the heart with the aid of the balloon and wherein the distance (11) between the balloons (4) is adjustable.

2. The catheter device according to claim 1, **characterized in that** the balloons are filled with gas and / or with liquid.

3. The catheter device according to claim 2, **characterized in that** the liquid is a coolant.

4. The catheter device according to claim 1, **characterized in that** shaft (15) comprises a hollow channel through which a further catheter can be inserted.

5. The catheter device according to any one of claims 1-4, **characterized in that** the catheter comprises at least one temperature sensor (5) which measures the temperature in the esophagus and optionally comprises a further temperature sensor (6).

6. The catheter device according to any one of claims 1-5, **characterized in that** the catheter comprises at least one magnet (12) on the side facing the heart.

7. The catheter device according to any one of claims 1-6, **characterized in that** at least one light-emitting diode LED (7) is arranged on shaft (15) parallel in the same electrode plane.

8. The multipolar esophageal electrode catheter device according to any one of claims 1-7, used for monitoring, stimulation, and cardioversion in cases of atrial fibrillation.

## Revendications

1. Multipôle cathéter d'électrode oesophagienne qui peut être utilisé pour la surveillance, la stimulation et la cardioversion dans le traitement de la fibrillation auriculaire du coeur, le cathéter ayant un tige flexible (15) avec une extrémité proximale et une extrémité distale, dans lequel l'extrémité distale du cathéter est conformée en ballon gonflable, ce ballon étant disposé d'un seul côté, **caractérisé en ce que** le cathéter comporte deux ballons gonflables (4), dans lequel une électrode multipolaire (3) est disposé sur chaque ballon de telle sorte que l'électrode multipolaire (3) peut être aligné approprié en direction du coeur à l'aide du ballon et **caractérisé en ce que** la distance (11) entre les ballons (4) est variable.

2. Le cathéter selon la revendication 1, **caractérisé en ce que** les ballons sont remplis avec du gaz et / ou avec du liquide.

3. Le cathéter selon la revendication 2, **caractérisé en ce que** le liquide est un coolant est.

4. Le cathéter selon la revendication 1, **caractérisé en ce que** le tige (15) comprend un canal creux à travers lequel un cathéter supplémentaire (14) peut être inséré.

5. Le cathéter selon l'une des revendications 1-4, **caractérisé en ce que** le cathéter comprend au moins un capteur de température (5) qui mesure la température dans l'oesophage, et que le catheter comprend éventuellement un autre capteur de température supplémentaire (6).

6. Le cathéter selon l'une des revendications 1-5, **caractérisé en ce que** le cathéter sur le côté faisant face à coeur comprend au moins un aimant (12).

7. Le cathéter selon l'une des revendications 1-6, **caractérisé en ce que** sur le tige (15) au moins une diode électroluminescente (LED) (7) est monté parallèle dans la même couche d'électrode.

8. Multipôle cathéter d'électrode oesophagienne selon l'une des revendications 1-7 pour la surveillance, la stimulation, et la cardioversion de la fibrillation auriculaire du coeur.
